(19) Europäisches Patentamt
European Patent Office
Office européen des brevets

(11) **EP 1 375 480 A1**

(12) # DEMANDE DE BREVET EUROPEEN

(43) Date de publication:
**02.01.2004 Bulletin 2004/01**

(21) Numéro de dépôt: **03291436.8**

(22) Date de dépôt: **13.06.2003**

(51) Int Cl.7: **C07D 207/12**, C07D 207/10, C07D 207/08, A61K 7/13, C07D 207/14, C07F 7/10

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **26.06.2002 FR 0207939**

(71) Demandeur: **L'OREAL**
**75008 Paris (FR)**

(72) Inventeurs:
- **Sabelle, Stéphane**
  **75005 Paris (FR)**
- **Ramos, Laure**
  **92340 Bourg Lareine (FR)**
- **Leduc, Madeleine, Rés. les Chèvrefeuilles**
  **75011 Paris (FR)**

(74) Mandataire: **Fevrier, Murielle**
**L'OREAL - D.I.P.I.**
**25-29 Quai Aulagnier**
**92600 Asnières (FR)**

(54) **Dérivés de para-phénylènediamine à groupement pyrrolidinyle substitué par un radical silyle et utilisation de ces dérives pour la coloration de fibres kératiniques**

(57) L'invention a pour objet une composition pour la teinture des fibres kératiniques, et en particulier des fibres kératiniques humaines telles que les cheveux, comprenant au moins un dérivé de para-phénylènediamine à groupement pyrrolidinyle substitué par un radical silylé, le procédé de teinture des fibres kératiniques mettant en oeuvre cette composition, ainsi que de nouveaux dérivés de para-phénylènediamine à groupement pyrrolidinyle substitué par un radical silylé.

La présente invention permet en particulier d'obtenir une coloration de fibres kératiniques chromatique, puissante, peu sélective et tenace.

EP 1 375 480 A1

## Description

L'invention a pour objet une composition pour la teinture des fibres kératiniques, et en particulier des fibres kératiniques humaines telles que les cheveux, comprenant au moins un dérivé de para-phénylènediamine à groupement pyrrolidinyle substitué par un radical silylé.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4-triazoles, des dérivés de pyrazolo [1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine, la para-toluènediamine sont des bases d'oxydation largement utilisées. Elles permettent d'obtenir avec des coupleurs d'oxydation des nuances variées.

Cependant, il existe un besoin de découvrir de nouvelles base d'oxydation présentant un meilleur profil toxicologique que la para-phénylènediamine et la para-toluènediamine, tout en permettant de conférer aux cheveux d'excellentes propriétés d'intensité de couleur, de variété de nuances, d'uniformité de la couleur et de ténacité aux agents extérieurs.

Il est déjà connu d'utiliser des dérivés de para-phénylènediamine subtitués par un groupement pyrrolidinique comme base d'oxydation pour la coloration de fibres kératiniques. Par exemple, le brevet US 5,851,237 décrit l'utilisation de dérivés 1-(4-aminophényl)pyrrolidine éventuellement substitués sur le noyau benzénique afin de remplacer la para-phénylènediamine.

Le brevet US 5,993,491 propose l'utilisation de dérivés de N-(4-aminophényl)-2-hydroxyméthylpyrrolidine éventuellement substitués sur le noyau benzènique et sur l'hétérocycle pyrrolidinique en position 4 par un radical hydroxy afin de remplacer la para-phénylènediamine.

La demande de brevet JP 11-158048 propose des compositions contenant au moins un composé choisi parmi des dérivés de 4-aminoaniline éventuellement substitués sur le noyau benzénique et dont un des atomes d'azote est compris dans un cycle de 5 à 7 chaînons carbonés.

Il est clairement établi que ces composés ne permettent pas de conférer aux cheveux une coloration de qualité équivalente à celle obtenue avec la para-phénylènediamine ou avec la para-toluènediamine du fait d'un manque d'intensité et d'uniformité de la couleur.

Il existe donc un réel besoin de découvrir de nouvelles bases d'oxydation présentant à la fois un bon profil toxicologique et des propriétés telles que les compositions les contenant permettent de conférer aux cheveux d'excellentes propriétés d'intensité de couleur, de variété de nuances, d'uniformité de la couleur et de ténacité vis à vis des différentes agressions extérieures que peuvent subir les cheveux.

Le but de la présente invention est de développer de nouvelles compositions tinctoriales ne présentant pas les inconvénients des bases d'oxydation de la technique antérieure en fournissant de nouvelles compositions tinctoriales pour la teinture de fibres kératiniques qui ne dégradent pas les fibres kératiniques, tout en étant capables d'engendrer des colorations intenses dans des nuances variées, peu sélectives particulièrement résistantes et présentant un bon profil toxicologique.

Ce but est atteint avec la présente invention qui a pour objet une composition pour la teinture des fibres kératiniques, et en particulier des fibres kératiniques humaines telles que les cheveux, comprenant, dans un milieu de

teinture approprié, au moins une base d'oxydation choisie parmi les dérivés de para-phénylènediamine substitués par un groupement pyrrolidinyle de formule (I) et leurs sels d'addition

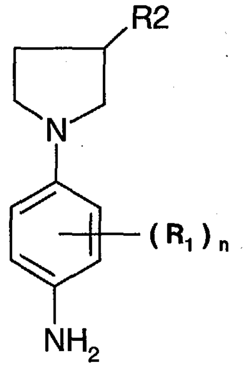

(I)

dans laquelle

- n est compris entre 0 et 4, étant entendu que lorsque n est supérieur ou égal à 2 alors les radicaux $R_1$ peuvent être identiques ou différents,
- $R_1$ représente un atome d'halogène ; une chaîne hydrocarbonée en $C_1$-$C_6$, aliphatique ou alicyclique, saturée ou insaturée, un ou plusieurs atomes de carbone de la chaîne hydrocarbonée pouvant être remplacés par un ou plusieurs atomes d'oxygène, d'azote, de silicium, de soufre, par un groupement $SO_2$ ; le radical $R_1$ ne comportant pas de liaison peroxyde, ni de radicaux diazo, nitro ou nitroso, cette chaîne pouvant être substituée par un ou plusieurs atomes d'halogène, un ou plusieurs radicaux hydroxyle, amino, mono- ou di-alkyl($C_1$-$C_4$)amino,
- $R_2$ représente

  - un radical $—SiR_3R_4R_5$,
  - un radical alkyle en $C_1$-$C_8$ pouvant être insaturé, linéaire ou ramifié, substitué par un radical $—SiR_3R_4R_5$ ; un ou plusieurs atomes de carbone du radical alkyle pouvant être remplaçés par un ou plusieurs atomes d'oxygène et/ou d'azote et le radical alkyle pouvant être substitué par un groupement hydroxyle, amino, alkyl($C_1$-$C_6$) amino ou dialkyl($C_1$-$C_6$)amino,
  - un radical triarylsilanyle, triarylsilanylalkyle, triarylsilanylalcoxy, triarylsilanylalkylamine, bis-(triarylsilanylalkyl) amine,

- $R_3$, $R_4$ et $R_5$, identiques ou différents, représentent un radical trialkyl($C_1$-$C_4$) silyle ; un radical triphénylsilyle; un radical phényle ; un radical alkyle en $C_1$-$C_6$ pouvant être substitué par un groupement trialkyl($C_1$-$C_6$)silyle, hydroxyle, amino, alkyl($C_1$-$C_6$)amino ou dialkyl($C_1$-$C_6$)amino.

L'invention a aussi pour objet de nouveaux dérivés de para-phénylènediamine à groupement pyrrolidinyle substitué par un radical silylé.
Un autre objet de l'invention est l'utilisation des dérivés de formule (I) pour la teinture de fibres kératiniques ainsi que le procédé de teinture de fibres kératiniques, en particuliers les fibres kératiniques humaines telles que les cheveux mettant en oeuvre la composition de la présente invention.
La composition de la présente invention permet en particulier d'obtenir une coloration de fibres kératiniques chromatique, puissante, peu sélective et tenace.
Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne ramifiée contenant une structure cyclique, pouvant contenir des insaturations du type alcène ou alcyne, mais ne contenant pas de structure cyclique aromatique.
Lorsque la chaîne est remplacée par un atome Y d'oxygène, de soufre, d'azote, de silicium ou $SO_2$, on obtient par exemple un motif $-CH_2-Y-CH_2-$.
A titre d'exemple, $R_1$ peut être un atome de chlore, un radical méthyle, éthyle, isopropyle, vinyle, allyle, méthoxyméthyle, hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1 -amino-2-hydroxyéthyle, 1,2-diaminoéthyle, méthoxy, éthoxy, allyloxy, 2-hydroxyéthyloxy.

Dans la formule (I), n est de préférence égal à 0 ou 1.

Selon un mode de réalisation particulier, $R_1$ est choisi parmi les atomes d'halogène par exemple le brome ou le chlore ; un radical alkyle en $C_1$-$C_4$, un radical hydroxyalkyle en $C_1$-$C_4$, un radical aminoalkyle en $C_1$-$C_4$, un radical alcoxy en $C_1$-$C_4$, un radical hydroxyalcoxy en $C_1$-$C_4$. A titre d'exemple, $R_1$ est choisi parmi un radical méthyle, isopropyle, hydroxyméthyle, 2-hydroxyéthyle, 1,2-dihydroxyéthyle, méthoxy, isopropyloxy, 2-hydroxyéthoxy.

Le radical $R_2$ est en particulier choisi parmi un radical trialkylsilanyle, trialkylsilanylalkyle, trialkylsilanylalcoxy, trialkylsilanylalkylamine, bis-(trialkylsilanylalkyl)amine, triarylsilanyle, triarylsilanylalkyle, triarylsilanylalcoxy, triarylsilanylalkylamine, bis-(triarylsilanylalkyl)amine. De préférence, $R_2$ est choisi parmi un radical trialkylsilanyle, trialkylsilanylalkyle, trialkylsilanylalcoxy, trialkylsilanylalkylamine, bis-(trialkylsilanylalkyl)amine.

Les radicaux $R_3$, $R_4$ et $R_5$ sont de préférence des radicaux alkyle, en particulier choisis parmi un radical méthyle, éthyle, propyle, isopropyle, butyle, isobutyle, terbutyle, pentyle, hexyle, cyclohexyle, ou des radicaux phényle ou toluyle

Les composés de formule (I) peuvent être sous forme de sels d'acide avec des acides minéraux forts tels que par exemple HCl, HBr, $H_2SO_4$, ou avec des acides organiques tels que, par exemple, l'acide acétique, lactique, tartrique, citrique ou succinique.

A titre d'exemples de dérivés de formule (I), on peut citer:

| | | | |
|---|---|---|---|
| 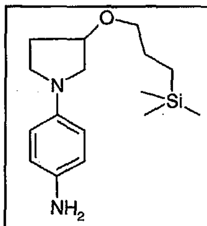 | 4-[3-(3-Triméthylsilanyl-propoxy)-pyrrolidin-1-yl]-phénylamine | 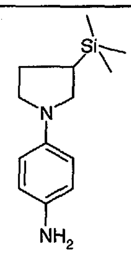 | 4-(3-Triméthylsilanyl-pyrrolidin-1-yl)-phénylamine |
| 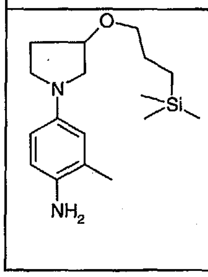 | 2-Méthyl-4-[3-(3-triméthylsilanyl-propoxy)-pyrrolidin-1-yl]-phénylamine | 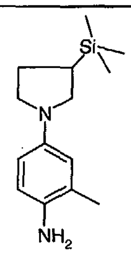 | 2-Méthyl-4-(3-triméthylsilanyl-pyrrolidin-1-yl)-phénylamine |

| | | | |
|---|---|---|---|
| 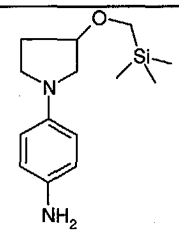 | 4-(3-Triméthylsilanylmethoxy-pyrrolidin-1-yl)-phénylamine | 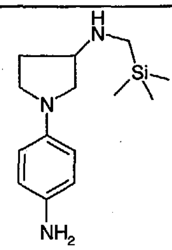 | [1-(4-Amino-phényl)-pyrrolidin-3-yl]-triméthylsilanylméthyl-amine |
| 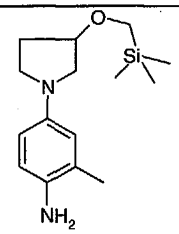 | 2-Méthyl-4-(3-triméthylsilanylmethoxy-pyrrolidin-1-yl)-phénylamine | 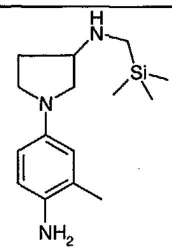 | [1-(4-Amino-3-méthyl-phényl)-pyrrolidin-3-yl]-triméthylsilanylméthyl-amine |
| 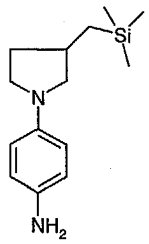 | 4-(3-Triméthylsilanylméthyl-pyrrolidin-1-yl)-phénylamine | 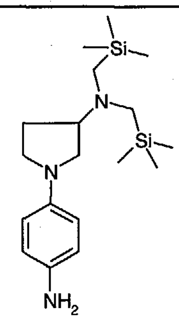 | [1-(4-Amino-phényl)-pyrrolidin-3-yl]-bis-triméthylsilanylméthyl-amine |
| 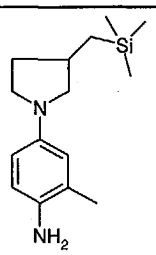 | 2-Méthyl-4-(3-triméthylsilanylméthyl-pyrrolidin-1-yl)-phénylamine | 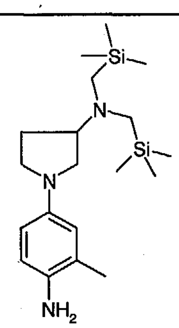 | [1-(4-Amino-3-méthyl-phényl)-pyrrolidin-3-yl]-bis-triméthylsilanylméthyl-amine |
| 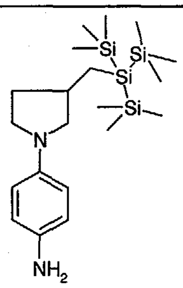 | 4-(3-Tri(triméthylsilanyl)silanylméthyl-pyrrolidin-1-yl)-phénylamine | 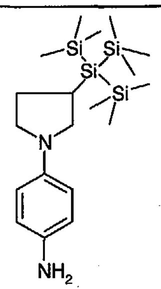 | 4-(3-Tri(triméthylsilanyl)silanyl-pyrrolidin-1-yl)-phénylamine |

| | | | |
|---|---|---|---|
| 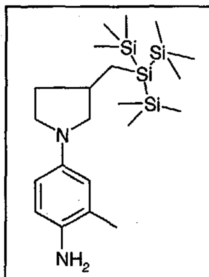 | 2-Méthyl-4-(3-tri(triméthylsilanyl)silanylméthyl-pyrrolidin-1-yl)-phénylamine | 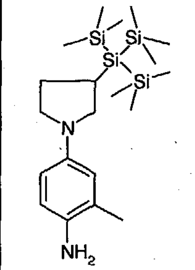 | 2-Méthyl-4-(3-tri(triméthylsilanyl)silanyl-pyrrolidin-1-yl)-phénylamine |
| 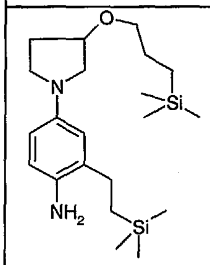 | 2-(2-Triméthylsilanyl-ethyl)-4-[3-(3-triméthylsilanyl-propoxy)-pyrrolidin-1-yl] phénylamine | 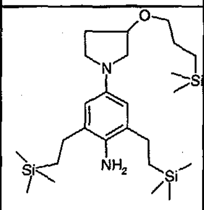 | 2,6-Bis-(2-triméthylsilanyl-ethyl)-4-[3-(3-triméthylsilanyl-propoxy)-pyrrolidin-1-yl] phényl amine |
| 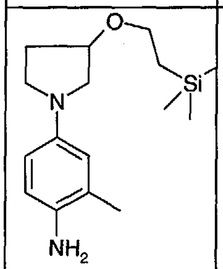 | 2-Méthyl-4-[3-(3-triméthylsilanyl-ethyloxy)-pyrrolidin-1-yl]-phénylamine | 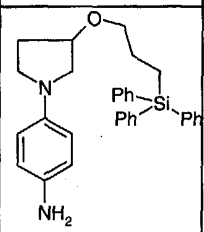 | 4-[3-(3-Triphenylsilanyl-propoxy)-pyrrolidin-1-yl]-phénylamine |
| 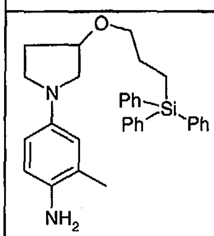 | 2-méthyl-4-[3-(3-Triphenylsilanyl-propoxy)-pyrrolidin-1-yl]-phénylamine | 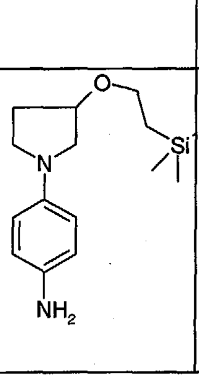 | 4-[3-(3-Triméthylsilanyl-ethyloxy)-pyrrolidin-1-yl]-phénylamine |

Parmi ces composés, les composés suivants sont particulièrement préférés

- 4-[3-(3-Triméthylsilanyl-propoxy)-pyrrolidin-1-yl]-phénylamine
- 4-(3-Triméthylsilanyl-pyrrolidin-1-yl)-phénylamine
- 4-(3-Triméthylsilanylmethoxy-pyrrolidin-1-yl)-phénylamine
- [1-(4-Amino-phenyl)-pyrrolidin-3-yl]-triméthylsilanylméthyl-amine
- 4-(3-Triméthylsilanylméthyl-pyrrolidin-1-yl)-phénylamine
- [1-(4-Amino-phenyl)-pyrrolidin-3-yl]-bis-triméthylsilanylméthyl-amine
- 2-(2-Triméthylsilanyl-éthyl)-4-[3-(3-triméthylsilanyl-propoxy)-pyrrolidin-1-yl] phénylamine

- 4-[3-(3-Triméthylsilanyl-ethyloxy)-pyrrolidin-1-yl]-phénylamine
- 4-[3-(3-Triphénylsilanyl-propoxy)-pyrrolidin-1-yl]-phénylamine.

Le carbone substitué par $R_2$ peut être de configuration R et / ou S.

La ou les bases d'oxydation de l'invention sont en général présentent chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(ß-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation. A titre d'exemple, ces bases d'oxydation additionnelles sont choisies parmi les para-phénylènediamines autres que celles décrites précédemment, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine; la 2-acétylamino pyrazolo-[1,5-a] pyridin-3-ylamine ; la 2-morpholin-4-yl-pyrazolo[1,5-a]pyridin-3-ylamine; l'acide 3-amino-pyrazolo[1,5-a]pyridin-2-carboxylique; la 2-méthoxy-pyrazolo[1,5-a]pyridine-3-ylamino ; le (3-amino-pyrazolo[1,5-a]pyridine-7-yl)-méthanol; le 2-(3-amino-pyrazolo [1,5-a]pyridine-5-yl)-éthanol; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine; la pyrazolo[1,5-a]pyridine-3,7-diamine ; la 7-morpholin-4-yl-pyrazolo[1,5-a]pyridin-3-ylamine; la pyrazolo[1,5-a]pyridine-3,5-diamine; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol; la 3-amino-pyrazolo[1,5-a] pyridine-5-ol ; 3-amino-pyrazolo[1,5-a]pyridine-4-ol; la 3-amino-pyrazolo[1,5-a]pyridine-6-ol ; la 3-amino-pyrazolo [1,5-a]pyridine-7-ol ; ainsi que leurs d'addition avec un acide ou avec une base.

Parmi les dérivés pyrimidiniques, on peut citer les composés décrits par exemple dans les brevets DE 2359399; JP 88-169571; JP 05-63124 ; EP 0770375 ou demande de brevet WO 96/15765 comme la 2,4,5,6-tétraaminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo [1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

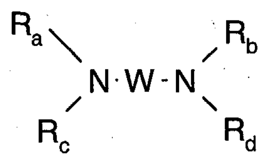

(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

L'invention a également pour objet les dérivés de para-phénylènediamine de formule (I) tels que définis précédemment, à l'exception du 5-amino-2-((3R)-3-t-butyldiméthylsilyloxy-1-pyrrolidinyl)fluorobenzène.

Les composés de formule (I) conformes à l'invention peuvent être facilement obtenus, selon des méthodes bien connues de l'état de la technique.

Les exemples qui suivent servent à illustrer l'invention sans toutefois présenter un caractère limitatif.

## EXEMPLES

**Exemple 1** : synthèse du 4-[3-(3-triméthylsilanyl-propoxy)-pyrrolidin-1-yl]-phénylamine: dihydrochlorure **(3)**

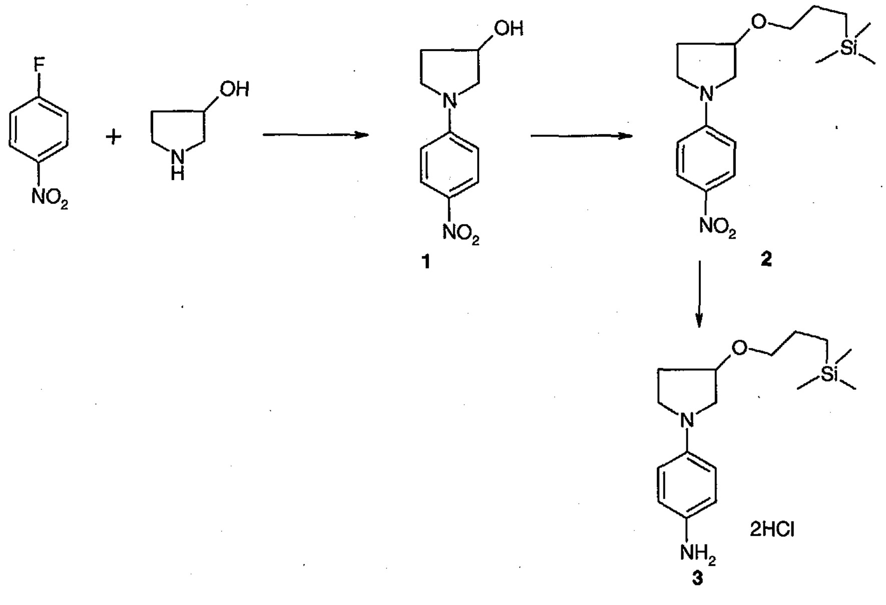

1 2 3

Synthèse du 1-(4-nitrophényl)-pyrrolidin-3-ol (**1**) :

Dans un tricol, on introduit 2 g de 1-fluoro-4-nitrobenzène (0.0155 mol), 1,3 g d'hydrogénocarbonate de sodium (0.0155 mol) et 15 ml d'un mélange dioxanne/eau (8/2). A ce mélange, on ajoute rapidement 1,35 g de 3-pyrrolidinol (0.0155 mol) racémique. Le mélange hétérogène est chauffé au reflux (87°C) pendant 10 heures. On verse ensuite le mélange réactionnel dans de l'eau glacée ; on obtient un précipité jaune que l'on filtre et rince à l'eau. Après séchage sous vide en présence de $P_2O_5$, 2,95 g d'un solide jaune sont obtenus (rendement 97%).

**RMN $^1$H** (DMSO $d_6$, 200 MHz, ppm) :

8,04 (d, J = 9 Hz, 2H); 6,58 (d, J = 9 Hz, 2H); 5,06 (d, J =3,6 Hz, 1H) ; 4,41 (m, 1H); 3,45 (m, 3H); 3,20 (m, 1H) ; 2,04

(m, 2H).

Synthèse de N-(4-nitrophényl)-3-(3-triméthylsilanyl-propoxy)-pyrrolidine (2)

0,504g (0,02 mmole) d'hydrure de sodium (60% dans l'huile) est agité dans 9 ml de diméthylformamide. 2,08g (0,01 mole) de N-(4-nitrophényl)-3-hydroxy-pyrrolidine (**1**) sont ajoutés au milieu réactionnel à température ambiante. Après 30 minutes d'agitation, 1,3g (0,0085 mole) de chloropropyltriméthylsilane sont ajoutés à température ambiante puis le milieu réactionnel est chauffé à 80°C pendant 5h30. Le mélange réactionnel est versé dans l'eau et le produit attendu est extrait au dichlorométhane. Les phases organiques sont évaporées sous pression réduite. Le résidu huileux ainsi obtenu est purifié par chromatographie sur gel de silice. On obtient 0,6g de N-(4-nitrophényl)-3-(3-triméthylsilanyl-propoxy)-pyrrolidine (2) sous forme d'une poudre jaune. Rendement=18%.
**Point de fusion =** 68°C
**RMN $^1$H** (400MHz, DMSO) ppm 8.05 (d, 2H), 6.63 (d, 2H), 4.21 (m, 1H), 3.46 (m, 6H), 2.09 (m, 2H), 1.47 (s, 2H), 0.45 (m, 2H), 0.04 (se, 9H)
**ESI+** : m/z=323(MH+), 345(MH+22)

Synthèse de 4-[3-(3-triméthylsilanyl-propoxy)-pyrrolidin-1-yl]-phénylamine; dichlorhydrate (**3**)

6g de zinc en poudre et 0,6g de chlorure d'ammonium sont chauffés au reflux dans 15 ml d'éthanol. 0,43g (0,00134 mole) du dérivé précédent **2** est ajouté lentement au mélange réactionnel. Le reflux est maintenu pendant 1h. On laisse revenir à température ambiante. Après filtration du zinc, 0,3 g de 4-[3-(3-triméthylsilanyl-propoxy)-pyrrolidin-1-yl]-phénylamine ; dichlorhydrate (**3**) est isolé sous forme de chlorhydrate. Rendement=61%
**RMN $^1$H** (400MHz, DMSO) ppm 9.95 (se, 2H), 7.18 (d, 2H), 6.58 (d, 2H), 4.17 (m, 1H), 3.31 (m, 6H), 2.06 (m, 2H), 1.47 (m, 2H), 0.46 (m, 2H), 0.03 (s, 9H)
**ESI+** : m/z= 292(M+)

**EXEMPLES DE TEINTURE EN MILIEU ALCALIN**

| Exemples | 1 | 2 | 3 | 4 |
|---|---|---|---|---|
| 4-[3-(3-Triméthylsilanyl-propoxy)-pyrrolidin-1-yl]-phénylamine (base) | $10^{-3}$ mole | $10^{-3}$ mole | $10^{-3}$ mole | $10^{-3}$ mole |
| 2-(2,4-Diamino-phénoxy)-éthanol, dichlorhydrate (coupleur) | 10-3 mole | | - | - |
| 3-Amino-2-chloro-6-méthyl-phénol, chlorhydrate (coupleur) | - | $10^{-3}$ mole | - | - |
| 3,6-Diméthyl-1H-pyrazolo[5,1-c] -[1,2,4]triazole (coupleur) | | - | $10^{-3}$ mole | |
| 2-Amino-pyridin-3-ol (coupleur) | - | - | - | $10^{-3}$ mole |
| Support de teinture (1) | (*) | (*) | (*) | (*) |
| Eau déminéralisée q.s.p. | 100 g | 100 g | 100 g | 100g |

(*) Support de teinture (1) pH 9,5

| | |
|---|---|
| Alcool éthylique à 96° | 20,8 g |
| Métabisulfite de sodium en solution aqueuse à 35% | 0,23g M.A |
| Sel pentasodique de l'acide diéthylène triamine-pentaacétique en solution aqueuse à 40 % | 0,48g M.A |
| Alkyl en $C_8$-$C_{10}$polyglucoside en solution aqueuse à 60% | 3,6g M.A |
| Alcool benzylique | 2,0g |
| Polyethylène glycol à 8 motifs d'oxyde d'éthylène | 3,0g |
| $NH_4Cl$ | 4,32g |
| Ammoniaque à 20% de $NH_3$ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| Exemples | 1 | 2 | 3 | 4 |
|---|---|---|---|---|
| **Nuance observée** | Bleu foncé | Violet bleu foncé | Violet rouge | Violet gris |

**EXEMPLES DE TEINTURE EN MILIEU ACIDE**

On a préparé les compositions tinctoriales suivantes :

| Exemples | 5 | 6 | 7 | 8 | 9 |
|---|---|---|---|---|---|
| 4-[3-(3-Triméthylsilanyl-propoxy)-pyrrolidin-1-yl]-phénylamine (base) | $10^{-3}$ mole | $10^{-3}$ mole | $10^{-3}$ mole | $10^{-3}$ mole | $10^{-3}$ mole |
| 2-(2,4-Diamino-phénoxy)-éthanol, dichlorhydrate (coupleur) | $10^{-3}$ mole | | - | - | |
| 3-Amino-2-chloro-6-méthyl-phénol, chlorhydrate (coupleur) | - | $10^{-3}$ mole | - | - | |
| 2-méthyl-5-aminophénol (coupleur) | - | - | $10^{-3}$ mole | - | |
| 2-Amino-pyridin-3-ol (coupleur) | - | - | - | $10^{-3}$ mole | |
| 6-Hydroxy-1-H-indole (coupleur) | - | | | - | $10^{-3}$ mole |
| Support de teinture (2) | (*) | (*) | (*) | (*) | (*) |
| Eau déminéralisée q.s.p. | 100g | 100 g | 100 g | 100 g | 100 g |

(*) Support de teinture (2) pH 7

| | |
|---|---|
| Alcool éthylique à 96° | 20,8 g |
| Métabisulfite de sodium en solution aqueuse à 35% | 0,23g M.A |
| Sel pentasodique de l'acide diéthylène triamine pentaacétique | 0,48g M.A |
| Alkyl en $C_8$-$C_{10}$polyglucoside en solution aqueuse à 60% | 3,6g M.A |
| Alcool benzylique | 2,0g |
| Polyéthylène glycol à 8 motifs d'oxyde d'éthylène | 3,0 g |
| $Na_2HPO_4$ | 0,28 g |
| $KH_2PO_4$ | 0, 46 g |

Au moment de l'emploi, chaque composition est mélangée avec un poids égal d'eau oxygénée à 20 volumes (6% en poids). On obtient un pH final de 7.
Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| Exemples | 5 | 6 | 7 | 8 | 9 |
|---|---|---|---|---|---|
| **Nuance observée** | Bleu violet foncé | Violet bleu foncé | Gris violet | Gris violet | Gris violet |

**Revendications**

1. Composition pour la teinture des fibres kératiniques comprenant, dans un milieu de teinture approprié, au moins une base d'oxydation choisie parmi les dérivés de para-phénylènediamine substitués par un groupement pyrrolidinyle de formule (I) et leurs sels d'addition

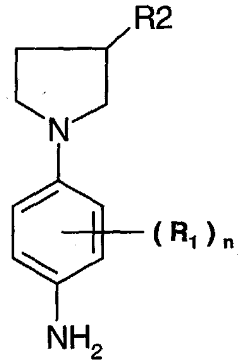

(I)

dans laquelle

- n est compris entre 0 et 4, étant entendu que lorsque n est supérieur ou égal à 2 alors les radicaux $R_1$ peuvent être identiques ou différents,

- $R_1$ représente un atome d'halogène ; une chaîne hydrocarbonée en $C_1$-$C_6$, aliphatique ou alicyclique, saturée ou insaturée, un ou plusieurs atomes de carbone de la chaîne hydrocarbonée pouvant être remplacés par un ou plusieurs atomes d'oxygène, d'azote, de silicium, de soufre, par un groupement $SO_2$ ; le radical $R_1$ ne comportant pas de liaison peroxyde, ni de radicaux diazo, nitro ou nitroso, cette chaîne pouvant être substituée par un ou plusieurs atomes d'halogène, un ou plusieurs radicaux hydroxyle, amino, mono- ou di-alkyl($C_1$-$C_4$) amino,

- $R_2$ représente

  - un radical $-SiR_3R_4R_5$ ,
  - un radical alkyle en $C_1$-$C_8$ pouvant être insaturé, linéaire ou ramifié, substitué par un radical $-SiR_3R_4R_5$ ; un ou plusieurs atomes de carbone du radical alkyle pouvant être remplaçés par un ou plusieurs atomes d'oxygène et/ou d'azote et le radical alkyle pouvant être substitué par un groupement hydroxyle, amino, alkyl($C_1$-$C_6$)amino ou dialkyl($C_1$-$C_6$)amino,
  - un radical triarylsilanyle, triarylsilanylalkyle, triarylsilanylalcoxy, triarylsilanylalkylamine, bis-(triarylsilanylalkyl)amine,

- R3, $R_4$ et $R_5$, identiques ou différents, représentent un radical trialkyl($C_1$-$C_4$) silyle ; un radical triphénylsilyle; un radical phényle ; un radical alkyle en $C_1$-$C_6$ pouvant être substitué par un groupement trialkyl($C_1$-$C_6$)silyle, hydroxyle, amino, alkyl($C_1$-$C_6$)amino ou dialkyl($C_1$-$C_6$)amino.

**2.** Composition selon la revendication 1 dans laquelle n est égal à 0 ou 1.

**3.** Composition selon la revendication 1 ou 2 dans laquelle $R_1$ est un atome d'halogène ; un radical alkyle en $C_1$-$C_4$, un radical hydroxyalkyle en $C_1$-$C_4$, un radical aminoalkyle en $C_1$-$C_4$, un radical alcoxy en $C_1$-$C_4$, un radical hydroxyalcoxy en $C_1$-$C_4$.

**4.** Composition selon la revendication 3 dans laquelle $R_1$ est choisi parmi un radical méthyle, isopropyle, hydroxyméthyle, 2-hydroxyéthyle, 1,2-dihydroxyéthyle, méthoxy, isopropyloxy, 2-hydroxyéthoxy.

**5.** Composition selon l'une quelconque des revendications 1 à 4 dans laquelle $R_2$ est un radical trialkylsilanyle, trialkylsilanylalkyle, trialkylsilanylalcoxy, trialkylsilanylalkylamine, bis-(trialkylsilanylalkyl)amine, triarylsilanyle, triarylsilanylalkyle, triarylsilanylalcoxy, triarylsilanylalkylamine, bis-(triarylsilanylalkyl)amine.

**6.** Composition selon la revendication 5 dans laquelle $R_2$ est un radical trialkylsilanyle, trialkylsilanylalkyle, trialkylsilanylalcoxy, trialkylsilanylalkylamine, bis-(trialkylsilanylalkyl)amine.

**7.** Composition selon l'une quelconque des revendications 1 à 6 dans laquelle $R_3$, $R_4$ et $R_5$ sont choisis parmi un radical méthyle, éthyle, propyle, isopropyle, butyle, isobutyle, terbutyle, pentyle, hexyle, cyclohexyle, phényle, toluyle.

**8.** Composition selon l'une quelconque des revendications 1 à 7 dans laquelle les dérivés de formule (I) sont choisis parmi
- 4-[3-(3-Triméthylsilanyl-propoxy)-pyrrolidin-1-yl]-phénylamine
- 4-(3-Triméthylsilanyl-pyrrolidin-1-yl)-phénylamine
- 4-(3-Triméthylsilanylmethoxy-pyrrolidin-1-yl)-phénylamine
- [1-(4-Amino-phenyl)-pyrrolidin-3-yl]-triméthylsilanylméthyl-amine
- 4-(3-Triméthylsilanylméthyl-pyrrolidin-1-yl)-phénylamine
- [1-(4-Amino-phényl)-pyrrolidin-3-yl]-bis-triméthylsilanylméthyl-amine
- 2-(2-Triméthylsilanyl-éthyl)-4-[3-(3-triméthylsilanyl-propoxy)-pyrrolidin-1-yl] phénylamine
- 4-[3-(3-Triméthylsilanyl-ethyloxy)-pyrrolidin-1-yl]-phénylamine
- 4-[3-(3-Triphenylsilanyl-propoxy)-pyrrolidin-1-yl]-phénylamine

**9.** Composition selon l'une quelconque des revendications précédentes comprenant de plus un coupleur choisi parmi les métaphénylènediamines, les méta-aminophénols, les métadiphénols, les coupleurs naphtaléniques, les coupleurs hétérocycliques et leurs sels d'addition

**10.** Composition selon la revendication 9 dans laquelle la quantité de chacun des coupleurs est comprise entre 0,001 et 10 % en poids environ du poids total de la composition tinctoriale.

**11.** Composition selon l'une quelconque des revendications précédentes comprenant une base d'oxydation additionnelle autre que les bases d'oxydation de formule (I) choisie parmi les para-phénylènediamines, les bis-phénylalkylènediamines, les para-aminophénols, les ortho-aminophénols, les bases hétérocycliques et leurs sels d'addition.

**12.** Composition selon l'une quelconque des revendications précédentes dans laquelle la quantité de chacune des bases d'oxydation est comprise entre 0,001 à 10 % en poids environ du poids total de la composition tinctoriale.

**13.** Composition selon l'une quelconque des revendications précédentes comprenant un agent oxydant.

**14.** Procédé de teinture d'oxydation des fibres kératiniques, **caractérisé en ce qu'**on applique sur les fibres une composition tinctoriale telle que définie dans l'une quelconque des revendications 1 à 12 en présence d'un agent oxydant pendant un temps suffisant pour développer la coloration désirée.

**15.** Procédé selon la revendication 14 dans lequel l'agent oxydant est choisi parmi le peroxyde d'hydrogène, le peroxyde d'urée, les bromates de métaux alcalins, les persels, les peracides et les enzymes oxydases.

**16.** Dispositif à plusieurs compartiments dans lequel un premier compartiment contient une composition tinctoriale telle que définie à l'une quelconque des revendications 1 à 12 et un deuxième compartiment contient un agent oxydant.

**17.** Utilisation des dérivés selon l'une quelconque des revendications 1 à 8 pour la teinture de fibres kératiniques.

**18.** Dérivés de para-phénylènediamine de formule (I) tels que définis à l'une quelconque des revendications 1 à 8, à l'exception du 5-amino-2-((3R)-3-*t*-butyldiméthylsilyloxy-1-pyrrolidinyl)fluorobenzène.

Office européen des brevets

# RAPPORT DE RECHERCHE EUROPEENNE

Numéro de la demande
EP 03 29 1436

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | JP 11 158048 A (FUJI PHOTO FILM CO LTD) 15 juin 1999 (1999-06-15)<br>* page 11; exemples A-29 *<br>-& US 2002/197223 A1 (FUJI PHOTO FILM CO LTD) 26 décembre 2002 (2002-12-26)<br>--- | 1,9 | C07D207/12<br>C07D207/10<br>C07D207/08<br>A61K7/13<br>C07D207/14<br>C07F7/10 |
| A | WO 01 68043 A (OREAL ;VIDAL LAURENT (FR); TERRANOVA ERIC (FR); SABELLE STEPHANE () 20 septembre 2001 (2001-09-20)<br>* exemples 1,2,5 *<br>* revendication 1 *<br>----- | 1,9 | |

| DOMAINES TECHNIQUES RECHERCHES (Int.Cl.7) |
|---|
| C07D<br>A61K<br>C07F |

Le présent rapport a été établi pour toutes les revendications

| Lieu de la recherche | Date d'achèvement de la recherche | Examinateur |
|---|---|---|
| LA HAYE | 2 octobre 2003 | Seitner, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 03 29 1436

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-10-2003

| Document brevet cité au rapport de recherche | Date de publication | Membre(s) de la famille de brevet(s) | Date de publication |
|---|---|---|---|
| JP 11158048 A | 15-06-1999 | US 2002197223 A1 | 26-12-2002 |
| WO 0168043 A | 20-09-2001 | FR 2806299 A1 | 21-09-2001 |
| | | AU 3938801 A | 24-09-2001 |
| | | BR 0105802 A | 26-03-2002 |
| | | CA 2373670 A1 | 20-09-2001 |
| | | CN 1372459 T | 02-10-2002 |
| | | EP 1200052 A2 | 02-05-2002 |
| | | WO 0168043 A2 | 20-09-2001 |
| | | HU 0201544 A2 | 28-08-2002 |
| | | US 2003093866 A1 | 22-05-2003 |

EPO FORM P0460

Pour tout renseignement concernant cette annexe : voir Journal Officiel de l'Office européen des brevets, No.12/82